(19) Europäisches Patentamt
European Patent Office
Office européen des brevets

(11) Publication number: **0 201 995 A2**

(12) **EUROPEAN PATENT APPLICATION**

(21) Application number: 86301892.5

(22) Date of filing: 14.03.86

(51) Int. Cl.[4]: C 07 C 67/42
C 07 C 69/612, C 07 C 69/734
C 07 C 69/65

(30) Priority: 15.03.85 JP 52965/85

(43) Date of publication of application:
20.11.86 Bulletin 86/47

(84) Designated Contracting States:
AT BE CH DE FR GB IT LI LU NL SE

(71) Applicant: Tamura, Yasumitsu
9-24 Sakasegawa 2-chome
Takarazuka Hyogo Pref.,(JP)

(72) Inventor: Tamura, Yasumitsu
9-24, Sakasegawa 2-chome
Takarazuka, Hyogo Pref.(JP)

(72) Inventor: Haruta, Junichi
13-11, Tezukayama Nishi 4-chome
Sumiyoshi-ku Osaka(JP)

(72) Inventor: Shirouchi, Yoshiaki
351, Kouzaki, Nanko-cho
Sayou-gun, Hyogo Pref.(JP)

(74) Representative: Eyles, Christopher Thomas et al,
BATCHELLOR, KIRK & EYLES 2 Pear Tree Court
Farringdon Road
London, EC1R 0DS(GB)

(54) **Method of preparing alpha-arylalkanoic esters.**

(57) Alpha-arylalkanoic esters are prepared by reacting a trivalent iodine compound of the formula

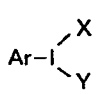

wherein Ar is an aromatic hydrocarbon and X and Y each represent a group which can be removed as an anion, with a carbonyl of the formula

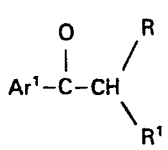

wherein $Ar^1$ is an aromatic hydrocarbon, R is a hydrogen atom or an alkyl group, and $R^1$ is a hydrogen atom or an alkyl group, in the presence of an orthocarboxylic ester having the general formula $ZC(OR^2)_3$, wherein $R^2$ is an alkyl group and Z is a hydrogen atom or an alkyl group; the reaction being carried out in the presence of at least one solvent selected from the group consisting of hydrocarbons, halogenated hydrocarbons, lower aliphatic esters, lower aliphatic ethers, lower aliphatic nitriles, lower aliphatic alcohols, lower aliphatic acids and nitroparaffins.

EP 0 201 995 A2

Croydon Printing Company Ltd.

# METHOD OF PREPARING α-ARYLALKANOIC ESTERS

## Technical Field

The present invention relates to a method of preparing α-arylalkanoic esters.

## Background of the Invention

Alpha-arylalkanoic acids are widely used as active anti-inflammatory, analgesic, and anti-pyretic pharmaceutical products. Such acids include, for example, ibuprofen, 2-(4-isobutylphenyl)propionic acid and fenoprofen, 2-(3-phenoxyphenyl)propionic acid. Various methods are known in the art for making these acids and their corresponding esters. For example, α-arylalkanoic esters can be made from corresponding carbonyl compounds of the general formula:

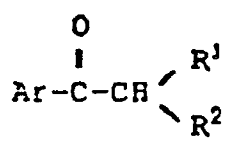

wherein at least one of the $R^1$ and $R^2$ groups is an alkyl group and the other is a hydrogen atom or an alkyl group or wherein $R^1$ is a bromine atom and $R^2$ is an alkyl group (Journal Am. Chem. Soc., 95:3340 [1973]; Synthesis, p. 126, [1981]; Synthesis, p. 456, [1982]; Perkin Transactions (British Chem. Soc.), 1:235 [1982]; Tetrahedron Letters, 23:235 [1982], Tetrahedron Letters 22:4305 [1981]; Journal Organic Chemistry, 43: 2936 [1978]; Chemical Communications, p. 1311, [1982]).

Each of the aforementioned methods has at least one disadvantage, such as requiring the use of a poisonous thallium or lead salt or a precious, and expensive,

silver salt, requiring a lengthy reaction time, and producing the desired product in low yields. Laid-Open Japanese Patent Publication No. 163,345 (1984), incorporated herein by reference, teaches a method of preparing α-arylalkanoic esters represented by the general formula

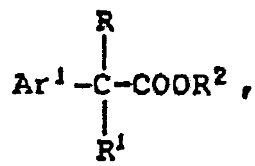

wherein $Ar^1$ is an aromatic hydrocarbon group, R and $R^1$ each represent a hydrogen atom or an alkyl group, and $R^2$ is an alkyl group, by reacting a compound of trivalent iodine having the general formula

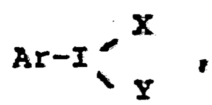

wherein Ar is an aromatic hydrocarbon gorup and X and Y are each a group which can be eliminated as an anion, with a carbonyl compound having the general formula

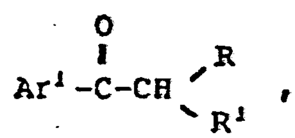

wherein $Ar^1$, R, and $R^1$ are as defined above. The reaction takes place in the presence of an orthocarboxylic ester having the general formula $ZC(OR^2)_3$, wherein $R^2$ is an alkyl group and Z is a hydrogen atom or an alkyl group. Although this method eliminates several disadvantages of methods taught by the prior art, it employs relatively large quantities of the orthocarboxylic ester, such as orthoformic methylester,

which can be an expensive reagent. Accordingly, further improvements are sought.

It thus is an object of the present invention to develop an economical method of preparing α-arylalkanoic ester compounds that is not hindered by the disadvantages of methods known in the art. Other objects of the present invention will become apparent by reading the description of the present invention contained herein.

## Summary of the Invention

In accordance with the present invention, α-arylalkanoic esters of the general formula

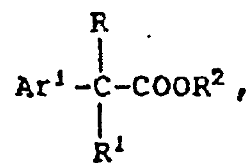

wherein $Ar^1$ is an aromatic hydrocarbon group, R and $R^1$ are each a hydrogen atom or an alkyl group, and $R^2$ is an alkyl group, are prepared by reacting a compound of trivalent iodine having the general formula

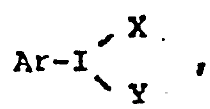

wherein Ar is an aromatic hydrocarbon group and X and Y are each a group which can be eliminated as an anion, with a carbonyl compound having the general formula

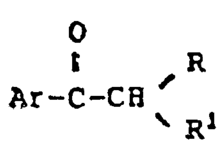

wherein $Ar^1$, R, and $R^1$ are as defined above, in the presence of an orthocarboxylic ester characterized by the

general formula $ZC(OR^2)_3$, wherein $R^2$ is an alkyl group and Z is a hydrogen atom or an alkyl group, in at least one solvent selected from the group consisting of hydrocarbons, halogenated hydrocarbons, lower aliphatic esters, lower aliphatic ethers, lower aliphatic nitriles, lower aliphatic alcohols, lower aliphatic acids, and nitroparaffins. By conducting the reaction in one of the aforementioned solvents, or a mixture thereof, a lesser amount of orthocarboxylic ester is needed than when the reaction is conducted in the absence of such a solvent.

## Detailed Description of the Invention

The present invention relates to a method of preparing α-arylalkanoic esters. In accordance with this method, the α-arylalkanoic esters are prepared by reacting a trivalent iodine compound with a carbonyl compound in the presence of an orthocarboxylic ester. Applicants have discovered that by conducting this reaction in the presence of one or more selected solvents, the amount of orthocarboxylic ester required for the reaction is decreased, thus improving the economies of the reaction process.

By the methods of this invention, α-arylalkanoic esters represented by the general formula

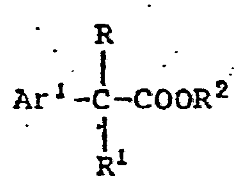,

where $Ar^1$ is an aromatic hydrocarbon, R is a hydrogen atom or an alkyl group, $R^1$ is a hydrogen atom or an alkyl group, and $R^2$ is an alkyl group, are prepared by reacting a trivalent-iodine compound represented by the general formula:

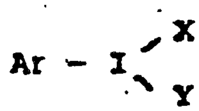,

wherein Ar is an aromatic hydrocarbon, and X and Y are each a group eliminated as an anion, with a carbonyl compound represented by the general formula:

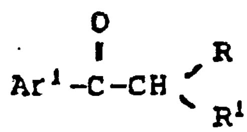

where $Ar^1$, R and $R^1$ are the same as defined above, in the presence of an orthocarboxylic ester represented by the general formula $ZC(OR^2)_3$, wherein $R^2$ is an alkyl group and Z is a hydrogen atom or an alkyl group, in at least one solvent selected from the group consisting of hydrocarbons, halogenated hydrocarbons, lower aliphatic esters, lower aliphatic ethers, lower aliphatic nitriles, lower aliphatic alcohols, lower aliphatic acids and nitroparaffins.

In this reaction process Ar represents an aromatic hydrocarbon group, which may have one or more substituents on the aromatic ring. The substituent may be a linear or branched alkyl group such as methyl, ethyl, n- or iso-propyl, or n-, iso-, sec- or t-butyl group, an alkoxy group such as methoxy, ethoxy, n- or iso-propyloxy, or n-, iso-, sec- or t-butoxy group, an aryloxy group such as phenoxy, an acyloxy group such as acetoxy, n- or iso-propionyloxy, n-, iso-, sec- or t-butyryloxy, or benzoyloxy group, or an electron attractive group such as a nitro, acetyl, propionyl, benzoyl, nitrile or sulfonyl group.

$Ar^1$ also represents an aromatic hydrocarbon group which optionally may carry a substituent on the aromatic ring. The substituent may be a saturated hydrocarbon

group such as an alkyl group having 1 to about 4 carbon atoms; an unsaturated aliphatic hydrocarbon group such as vinyl, ethynyl, or allyl group, an alkenyl or alkenyloxy group having such an unsaturated group; an alkoxy group such as methoxy, ethoxy, n- or iso-propyloxy, or n-, iso-, sec-, or t-butyloxy; an alkylthio group such as methylthio, ethylthio, n- or iso-propylthio, or n-, sec-, iso- or t-butylthio group; an arylthio group such as phenylthio; an aryl group, such as phenyl; a halogen atom or an amino group which is mono- or di-substituted by n- or iso-propyl, or n-, iso-, sec- or t-butyl group.

The groups R and $R^1$ may each independently represent an alkyl group, such as methyl, ethyl or propyl, or a hydrogen atom.

Preferably, the carbonyl compound is an acetophenone or propiophenone, the phenyl group of which optionally is substituted with an alkyl group, halogen or alkoxy group. The ratio of trivalent iodine compound to carbonyl compound desirably is at least 1:1. A desirable ratio is about 1.2:1.

In trivalent-iodine compounds $Ar{-}I{<}^{X}_{Y}$, X and Y are groups which can be eliminated as an anion and include, for example, acyloxy groups such as acetoxy, trifluoroacetoxy, benzoyloxy, chloro and fluoro. X and Y may be the same group or different groups and may include a combination of an acyloxy group as X and a hydroxy group as Y.

The trivalent-iodine compounds can be produced in accordance with procedures well known in the art. For example, if X and Y are chlorine atoms, $Ar{-}I{<}^{Cl}_{Cl}$ can be prepared by reacting an iodinated aromatic hydrocarbon, Ar-I, with chlorine. If the dichloro trivalent-iodine

compound obtained is allowed to react with acetic acid, the chlorine can be replaced with an acetoxy group. In the same way other trivalent-iodine compounds having other electronegative groups also can be produced.

The reaction is carried out in the presence of an orthocarboxylic ester represented by the general formula $ZC(OR^2)_3$. In this formula Z is a hydrogen atom or an alkyl group and $R^2$ is an alkyl group. Preferably, the compound is the methyl, ethyl or propyl ester of ortho-formic acid. Ethyl orthoformate is especially preferred in comparison to other orthocarboxylic esters in that it is the most economical.

To obtain the desired α-arylalkanoic esters, the trivalent iodine compound and carbonyl compound are reacted together in the presence of the orthocarboxylic ester in at least one of a selected group of solvents. The solvent(s) are selected from the group consisting of hydrocarbons, halogenated hydrocarbons, lower aliphatic esters, lower aliphatic ethers, lower aliphatic nitriles, lower aliphatic alcohols, lower aliphatic acids and nitroparaffins. "Lower" is defined herein to include compounds having about 5 or fewer carbon atoms.

~~Applicants~~ The inventors have found that when the reaction is carried out in one of these selected solvents the amount of orthocarboxylic acid needed is significantly decreased in comparison to the amount required when the reaction is carried out in the absence of such a solvent. In the example of Japanese Laid Open Patent Application 163,345, the molar ratio of orthocarboxylic ester to carbonyl compound was shown to be about 14:1. In accordance with the method of this invention, the molar ratio of orthocarboxylic ester to carbonyl compound can be as low as about 1:1. If desired, additional orthocarboxylic ester, sufficent to bring the amount to about three or more equivalents of the amount of carbonyl can be

employed. Typically, no more than 6-8 equivalents of orthocarboxylic ester are needed to obtain high yields of product.

The solvent used in the present invention is selected from specific groups as mentioned above. Examples of the solvents include linear or cyclic hydrocarbons having about 5 to about 7 carbon atoms, such as n-hexane, cyclopentane, cyclohexane, benzene and toluene; linear or cyclic halogenated hydrocarbons having 1 to about 6 carbon atoms, such as chloroform, dichloromethane and chlorobenzene; lower alkylesters such as methyl, ethyl and propyl esters of a fatty acid having 1 to about 3 carbon atoms, such as formic acid, acetic acid and propionic acid; lower aliphatic ethers having about 2 to about 4 carbon atoms such as dimethyl ether, diethyl ether and methyl ethyl ether; lower aliphatic nitriles, such as acetonitrile and propionitrile; lower aliphatic alcohols having 1 to about 4 carbon atoms, including methanol, ethanol, n- or iso-propanol, or t-butanol; lower fatty acids having 1 to about 3 carbon atoms, i.e., formic acid, acetic acid and propionic acid; and nitroparaffins having 1 to about 2 carbon atoms, such as nitromethane and nitroethane. A preferred solvent is ethyl acetate.

The solvents may be used singly or as a mixture of two or more. If solvent recovery is taken into consideration, use of a single solvent may be preferable for ease of recovery of the reaction product.

In the present invention, beneficial results are obtained when the solvent is selected from the list set forth above. It has been found, however, as illustrated in the examples below, that if other solvents, such as a lower aliphatic ketone such as acetone, an amide of a lower fatty acid di-substituted with lower alkyl groups such as dimethyl formamide, or lower dialkyl sulfoxide

such as dimethyl sulfoxide is used as a solvent, the reaction of the present invention will not proceed substantially and the reactant which remains unreacted will be recovered or the product will be resinified.

The reaction of the present invention desirably is carried out under acid conditions as, for example, by adding concentrated sulfuric acid or other acid such as perchloric acid. The reaction may be carried out at room temperature. Alternatively, if desired, heat may be applied to raise the reaction temperature up to about 80°C.

Reaction time is dependent upon the carbonyl compound chosen as a reactant. The completion of the reaction can be determined by thin layer chromatography (TLC) by measuring for the disapperance of carbonyl compound. The α-arylalkanoic ester produced by the reaction can be recovered from the reaction mixture in accordance with conventional methods.

The present invention is further illustrated by the following examples, which are provided for illustrative purposes only and are not to be construed as limiting.

## Example I

A series of reactions was run to determine the effect of various solvents on the production of α-arylalkanoic esters.

A mixture of one millimol (134 mg) of propiophenone, 1.2 millimole (386 mg) of iodobenzene diacetate and 6 millimol (0.65 ml) of methyl orthoformate was stirred at room temperature for 3 hours in the presence of 2 millimol of concentrated sulfuric acid in 3 ml of one of the solvents listed in Table I below. The reaction mixture obtained was extracted with benzene, chloroform, ethyl acetate or diethyl ether. The extract was washed

with water and dried with anhydrous magnesium sulfate and the solvent was distilled off. The residue was purified by means of silicagel column chromatography. The results are shown in Table 1.

The infrared spectrum and nuclear resonance spectra of the resulting product compound were as follows:

$IR\nu_{max}^{CHCl_3}$ $cm^{-1}$: 1730

$^{1}H$-NMR ($CDCl_3$)δ 1.48 (d, J=7Hz, 3H); 3.62 (s, 3H); 3.70 (q, J=7Hz, 1H); 7.24 (s,5H)

<u>Table 1</u>

| Solvent for Reaction | Solvent for Extraction | Product Yield (%) |
|---|---|---|
| n-hexane | Ether | 81 |
| Benzene | Benzene | 86 |
| Chloroform | Chloroform | 85 |
| Ethylacetate | Ethylacetate | 82 |
| Diethyl ether | Diethyl ether | 83 |
| Acetonitrile | Diethyl ether | 82 |
| Nitromethane | Diethyl ether | 68 |
| Acetic acid | Diethyl ether | 66 |
| Methanol | Diethyl ether | 64 |
| Acetone | Diethyl ether | 0 |
| Dimethyl formamide | Diethyl ether | 0 |
| Dimethyl sulfoxide | Diethyl ether | 0 |

As illustrated in Table I, when the reaction was carried out in the presence of acetone, dimethyl formamide or dimethyl sulfoxide as solvent, no measurable amount of desired product compound was obtained. When the reaction was carried out in the presence of any of the other solvents, however, the product yield was at least 64%.

## Example 2

The procedure of Example 1 was followed with the exception that ethyl orthoformate was used in place of methyl orthoformate, 2.5 ml. of ethyl acetate was used as the solvent, and the amount of concentrated $H_2SO_4$ was changed to 1.5 millimol. Ethyl acetate was used as the solvent for extraction.

The results are shown in Table 2.

The infrared spectrum and nuclear resonance spectra of the product compound were as follows:

IR $\nu_{max}^{CHCl_3}$ $cm^{-1}$: 1725

$^1H$-NMR ($CDCl_3$)δ: 1.21 (t, J=7Hz, 3H);
1.48 (d, J=7Hz, 2H);
3.68 (q, J=7Hz, 1H);
4.10 (q, J=7Hz, 2H);
7.26 (s, 5H)

Table 2

| $HC(OEt)_3$ millimol | Time hr | Product Yield % |
|---|---|---|
| 12 | 1.5 | 91 |
| 10 | 1.5 | 88 |
| 8 | 2 | 84 |
| 6 | 3 | 82 |
| 4 | 6 | 78 |
| 3 | 7 | 71 |
| 2 | 14 | 52 |
| 1 | 24 | 30 |

The data in Table 2 show that when the reaction is carried out in ethyl acetate as the solvent, significant amounts of product can be obtained even when the ratio of orthocarboxylic ester to carbonyl compound is approximately 1:1.

## Example 3

The procedure of Example 1 was followed with the exception that varying amounts of methyl orthoformate were used and 3 ml. of ethyl acetate were used as the solvent. Ethyl acetate also was used as the solvent for extraction. The results are shown in Table 3.

Table 3

| $HC(OMe)_3$ millimol | Time hr | Product Yield % |
|---|---|---|
| 12 | 1.5 | 92 |
| 10 | 1.5 | 88 |
| 8 | 2 | 86 |
| 6 | 3 | 82 |
| 3 | 4 | 80 |
| 2 | 5 | 62 |
| 1 | 14 | 51 |

The data in Table 3 show that when the reaction is carried out in ethyl acetate as the solvent, significant amounts of product can be obtained even when the ratio of orthocarboxylic ester to carbonyl compound is approximately 1:1.

## Example 4

Selected arylpropionic methyl esters were prepared as follows:

A mixture of 1 millimol of an arylethyl ketone (the aryl group of each compound is illustrated in column 1 of Table 4), 1.2 millimol of iodobenzene diacetate and 3 millimol of methyl orthoformate was stirred in 3 ml of ethyl acetate at temperatures of from room temperature to 50°C in the presence of 2 millimol of concentrated $H_2SO_4$. The reaction mixture was extracted with ethyl acetate and the extract was washed with water and dried with $MgSO_4$.

The solvent was distilled off, and the residue was purified with silicagel column chromatography. The results are shown in Table 4.

Table 4

| Ar | Reaction Time Hour | Product Yield (%) |
|---|---|---|
| 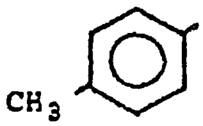 | 3 | 83 |
| 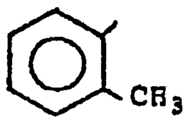 | 4 | 80 |
| 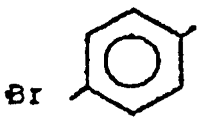 | 10 | 78 |
| 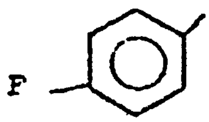 | 8 | 81 |
| 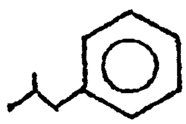 | 3 | 82 |

As illustrated in Table 4, a high yield of product was obtained from each carbonyl compound starting material.

Example 5

Selected arylacetic methyl esters were prepared as follows:

The procedure of Example 4 was followed with the exception that one of the starting materials was an arylmethyl ketone rather than an arylethyl ketone. The results are shown in Table 5. The aryl portion of each arylmethylketone selected is illustrated in the first column of the table.

## Table 5

| Ar | Reaction Time Hour | Product Yield (%) |
| --- | --- | --- |
| 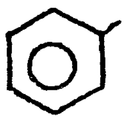 | 4 | 71 |
| 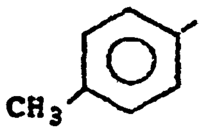 | 4 | 68 |
| 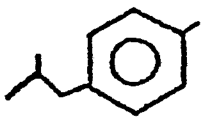 | 4 | 70 |
| 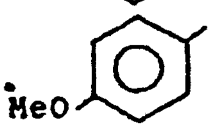 | 2 | 73 |
| 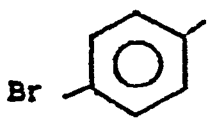 | 5 | 53 |

As illustrated in Table 5, a high yield of product was obtained from each carbonyl compound starting material.

## Claims

1. A method for preparing an α-arylalkanoic ester represented by the general formula

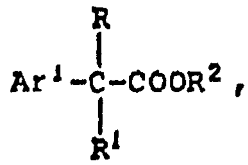

wherein $Ar^1$ is an aromatic hydrocarbon, R is an alkyl group or a hydrogen atom, $R^1$ is an alkyl group or a hydrogen atom, and $R^2$ is an alkyl group, which comprises reacting, under α-arylalkanoic ester-producing conditions, a trivalent iodine compound represented by the general formula

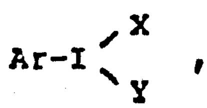

wherein Ar is an aromatic hydrocarbon and X and Y each represents a group which can be eliminated as an anion, with a carbonyl compound represented by the general formula

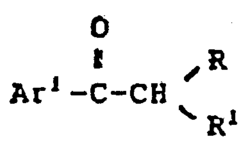

wherein $Ar^1$, R and $R^1$ are as defined above, in the presence of an orthocarboxylic ester represented by the general formula

$$ZC(OR^2)_3$$

wherein Z is an alkyl group or a hydrogen atom and $R^2$ is as defined above, in the presence of at least one solvent selected from the group consisting of hydrocarbons, halogenated hydrocarbons, lower aliphatic esters, lower aliphatic ethers, lower aliphatic alcohols, lower

aliphatic nitriles, lower aliphatic acids and nitroparaffins.

2. The method of claim 1 wherein said solvent or solvents is selected from the group consisting of a linear or cyclic hydrocarbon comprising 5 to about 7 carbon atoms; a linear or cyclic halogenated hydrocarbon comprising 1 to about 6 carbon atoms; methyl, ethyl, n- or isopropyl ester of a fatty acid comprising 1 to about 3 carbon atoms; an aliphatic ether comprising about 2 to about 4 carbon atoms; an alkyl cyanide comprising 1 to about 2 carbon atoms; an aliphatic alcohol comprising 1 to about 4 carbon atoms; a fatty acid comprising 1 to about 3 carbon atoms and nitrated paraffins comprising 1 to about 2 carbon atoms.

3. The method of claim 1 or 2 wherein Ar is a phenyl group which is optionally substituted by an alkyl, alkoxy, aryloxy; acyloxy or electron attractive group, X and Y are each a halogen atom or an aliphatic or aromatic acyloxy group; $Ar^1$ is a phenyl group which is optionally substituted by a saturated or unsaturated hydrocarbon, aryl, alkoxy, alkenyloxy, alkynyloxy, alkylthio, substituted amino group or a halogen atom; and R and $R^1$ are each a hydrogen atom or an alkyl group comprising one to three carbon atoms.

4. A method as claimed in claim 1 or 2 where Ar is a phenyl group which optionally carries a substituent comprising an alkyl group having 1 to about 4 carbon atoms, an alkoxy group comprising 1 to about 4 carbon atoms, a phenyloxy group, an acyloxy group comprising 1 to about 4 carbon atoms, or a nitro, acyl, cyano, or sulfonyl group; X and Y each comprise a halogen atom or an aliphatic acyloxy group comprising about three or fewer carbon atoms; $Ar^1$ is a phenyl group which optionally carries a substituent comprising an alkyl group comprising 1 to about 4 carbon atoms, a phenyl

group, an alkoxy group comprising 1 to 4 carbon atoms, a phenoxy group, an aliphatic acyloxy group, benzoyloxy group, alkylthio group comprising 1 to about 4 carbon atoms, or mono- or di-substituted alkyl or phenyl amino group or a halogen atom; and R and $R^1$ each comprises a hydrogen atom or an alkyl group comprising 1 to 3 carbon atoms.

5. The method of claim 1 or 2 wherein said orthocarboxylic ester comprises methyl orthoformate, ethyl orthoformate, or propyl orthoformate.

6. The method of claim 1 wherein the molar ratio of said orthocarboxylic ester to said carbonyl compound is about 1:1 to about 8:1.

7. The method of claim 1 wherein the ratio of trivalent iodine compound to carbonyl compound is at least 1:1 and the reaction is conducted at room temperature.

8. The method of claim 1 wherein the reaction is conducted in the presence of concentrated sulfuric acid.